# FASCICULE DE BREVET EUROPEEN

(11) **EP 0 659 399 B1**
(45) Date de publication et mention de la délivrance du brevet: **19.03.1997**
(21) Numéro de dépôt: 94402572.5
(22) Date de dépôt: 14.11.1994
(51) Int. Cl.: A61K 7/13, A45D 7/00, A45D 19/00

(54) **Procédé de coloration directe des fibres kératiniques humaines à l'aide de vapeur d'eau**
Verfahren zum direkten Färben von Menschlichen Keratinfasern mit Wasserdampf
Process of direct hair using steam

(30) Priorité: 22.12.1993 FR 9315485
(43) Date de publication de la demande: 28.06.1995
(73) Titulaire: L'OREAL, 75008 Paris (FR)
(72) Inventeur: Samain, Henri, F-91570 Bievres (FR); Sturla, Jean-Michel, F-92210 Saint-Cloud (FR)
(74) Mandataire: Andral, Christophe André Louis

(56) Documents cités:
- EP-A- 0 103 547
- EP-A- 0 312 343
- FR-A- 2 273 492
- GB-A- 2 168 082

## Description

La présente invention est relative à un procédé de coloration (ou teinture) directe des fibres kératiniques, en particulier les cheveux, mettant en oeuvre de la vapeur d'eau et une composition comprenant des colorants directs particuliers.

Il est bien connu dans le domaine de la coloration capillaire d'utiliser des colorants dits d'oxydation qui conduisent à des nuances très couvrantes et tenaces. La coloration d'oxydation nécessite toutefois la mise en oeuvre d'agents oxydants qui peuvent à la longue abîmer les cheveux lorsque de telles teintures sont appliquées très fréquemment.

Aussi dans le but de réaliser des colorations certes moins tenaces mais par ailleurs moins dégradantes pour le cheveu, on a utilisé des colorants dits directs, c'est à dire des colorants qui ne mettent pas en oeuvre un mécanisme d'oxydation et qui sont capables de colorer par eux-mêmes les fibres kératiniques.

De tels colorants, grâce à la variété des substances qu'il est possible de mettre en oeuvre, permettent de couvrir une large gamme de nuances allant du jaune au bleu en passant par le rouge.

Parmi les colorants directs, on peut citer les colorants benzéniques nitrés qui sont performants et généralement bien tolérés.

En coloration capillaire, les teintes bleues sont nécessaires comme composantes permettant d'arriver aux teintes d'aspect naturel, et l'on a déjà proposé d'employer comme colorants directs capillaires bleus, les dérivés de la nitro-2 paraphénylènediamine dont le groupe amino en position 4 est disubstitué, le groupe amino en position 1 étant quant à lui monosubstitué. Pour obtenir des nuances à reflets, on utilise particulièrement les dérivés nitrés des aminophénols.

Ces deux classes de colorants présentent cependant l'inconvénient d'être très sélectifs au niveau de la fibre à colorer.

On appelle sélectivité d'un colorant la différence de montée (i.e de pouvoir de coloration) de celui-ci sur la fibre capillaire selon que celle-ci a été plus ou moins sensibilisée (i.e "abîmée") soit par un traitement tel qu'une décoloration ou une permanente, soit par les agents atmosphériques notamment dans le cas des pointes des cheveux.

Ainsi, d'une façon générale, les colorants directs ci-dessus prennent mieux sur des cheveux légèrement sensibilisées que sur des cheveux naturels.

Ce problème de sélectivité a pour conséquence de déséquilibrer les teintes ou les reflets suivant le degré de sensibilisation (i.e l'état de dégradation) du cheveu sur lequel on applique la composition contenant les colorants.

Les résultats de coloration obtenus sur des cheveux présentant des différences de sensibilisation sont donc hétérogènes. Ces irrégularités ne sont bien évidemment pas souhaitables d'un point de vue esthétique.

La présente invention vise à résoudre le problème ci-dessus.

La demanderesse a maintenant découvert de façon surprenante que l'utilisation d'un gaz chauffé à une température supérieure à 75°C comprenant de la vapeur d'eau sur des cheveux traités avec certains colorants directs, permettait d'obtenir des résultats tinctoriaux dépendant peu ou pas du tout du degré de sensibilisation des fibres kératiniques à colorer.

Selon l'invention, les cheveux sont ainsi colorés de façon uniforme sur l'ensemble de la chevelure, des racines aux pointes, et ceci quel que soit l'état du cheveu.

On notera que l'utilisation de la vapeur d'eau dans divers procédés de traitements capillaires a déjà été décrite dans la demande de brevet FR-A-2273492, document dans lequel de la vapeur d'eau chauffée à environ 100-150°C est mise en oeuvre notamment dans le but d'accélérer ou de provoquer des réactions chimiques de composés qui sont déjà placés sur la chevelure. Un exemple de coloration capillaire directe décrit ainsi l'utilisation de la 2-nitro paraphénylènediamine. Toutefois, la sélectivité (au sens défini ci-avant) de ce colorant n'est pas diminuée lorsque l'on emploie un procédé à la vapeur d'eau, et un tel colorant est exclu du cadre de la présente invention.

La présente invention concerne ainsi un procédé de teinture des fibres kératiniques, caractérisé par le fait qu'il consiste à mettre en contact des fibres kératiniques sur lesquelles on a appliqué une composition contenant au moins un colorant direct choisi parmi les dérivés de la 2-nitroparaphénylènediamine de formule (I) ci-dessous et/ou les dérivés nitrés des aminophénols de formule (II) ci-dessous, avec un gaz contenant de la vapeur d'eau, la température dudit gaz étant supérieure à 75°C, le temps de contact entre ledit gaz et lesdites fibres à colorer étant inférieur à deux minutes.

Les colorants directs adaptés à l'invention répondent aux formules (I) et (II) suivantes : 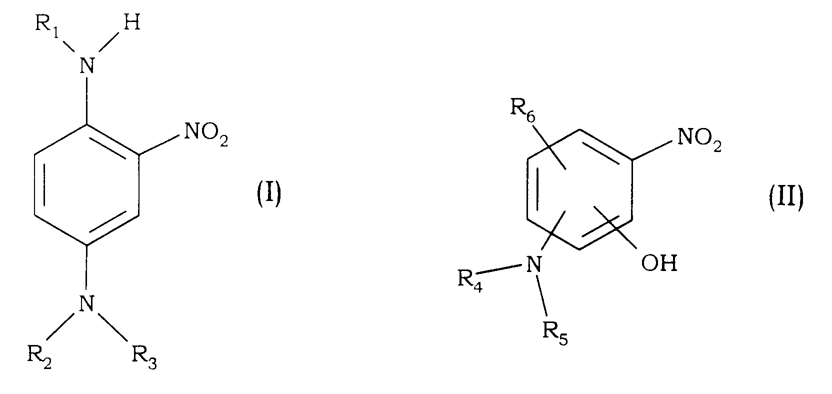 dans lesquelles :
R₁ est choisi parmi les radicaux alkyle en C₁-C₄, monohydroxyalkyle en C₂-C₄, polyhydroxyalkyle en C₂-C₄, les radicaux alkoxy(C₁-C₄)alkyle(C₁-C₄) et aminoalkyle en C₁-C₄ ;
R₂ et R₃ sont choisis parmi les radicaux alkyle en C₁-C₄, monohydroxyalkyle en C₂-C₄, polyhydroxyalkyle en C₂-C₄ ;
R₄ et R₅ sont choisis parmi l'hydrogène, les radicaux alkyle en C₁-C₄, monohydroxyalkyle en C₂-C₄, polyhydroxyalkyle en C₂-C₄ et aminoalkyle en C₁-C₄ ;
R₆ est choisi parmi l'hydrogène, les radicaux alkyle en C₁-C₄, les atomes d'halogène et le groupement NO₂.

Les colorants de formule (I) sont choisis de préférence parmi les composés suivants :
N,N',N'-tris(2'-hydroxyéthyl) 1,4-diamino 2-nitro benzène
1-méthylamino 2-nitro 4-bis(2'-hydroxyéthyl)amino benzène
1-méthylamino 2-nitro 4-(N-méthyl N-2'-hydroxyéthyl)amino benzène
1-(2'-aminoéthyl)amino 2-nitro 4-bis(2'-hydroxyéthyl)amino benzène
4-(N-éthyl N-2'-hydroxyéthyl)amino 1-(2'-hydroxyéthyl)amino 2-nitro benzène
1 -(2'-méthoxyéthyl)amino 2-nitro 4-bis(2'-hydroxyéthyl)amino benzène
1-(2',3'-dihydroxypropyl)amino 2-nitro 4-bis(2'-hydroxyéthyl)amino benzène
1-(2',3'-dihydroxypropyl)amino 2-nitro 4-(N-méthyl N-2'-hydroxyéthyl)amino benzène
1-(2',3'-dihydroxypropyl)amino 2-nitro 4-(N-éthyl N-2'-hydroxyéthyl)amino benzène
1-méthylamino 2-nitro 4-(N-méthyl N-2',3'-dihydroxypropyl)amino benzène.

Les colorants de formule (II) sont choisis de préférence parmi les composés suivants :
3-amino 4-hydroxy nitrobenzène
3-hydroxy 4-amino nitrobenzène
2-hydroxy 3-amino 1,5-dinitrobenzène
2-hydroxy 5-amino nitrobenzène
1-hydroxy 3-nitro 4-N-(2'-hydroxyéthyl)amino benzène
1-hydroxy 2-amino 3-nitro benzène
1-amino 2-nitro 4-hydroxy 5-méthyl benzène
1-N-(2'-hydroxyéthyl)amino 2-hydroxy 4-nitro benzène
1-hydroxy 2-N-(2'-hydroxyéthyl)amino 4,6-dinitrobenzène
2-chloro 6-N-éthylamino 1-hydroxy 4-nitro benzène
6-chloro 4-nitro 2-amino phénol
1-hydroxy 3-nitro 4-N-(3'-hydroxypropyl)amino benzène
1-hydroxy 2-N,N-(2'-hydroxyéthyl)amino 5-nitro benzène
2-amino 4-hydroxy 1-nitro benzène
2-hydroxy 4-amino 1-nitro benzène
1-hydroxy 3-nitro 4-amino benzène
2-hydroxy 5-N-(2'-hydroxyéthyl)amino 1-nitro benzène
2-hydroxy 6-amino 1-nitro benzène
2-hydroxy 3-amino 1-nitro benzène
2-hydroxy 3-chloro 6-amino 1-nitro benzène
2-N-(2'-hydroxyéthyl)amino 3-hydroxy 1-nitro benzène
3-N-(2'-hydroxyéthyl)amino 4-hydroxy 1-nitro benzène
2-hydroxy 6-N-(2'-hydroxyéthyl)amino 1-nitro benzène
1-hydroxy 2,6-dinitro 4-méthylamino benzène
2-N-(2'aminoéthyl)amino 4-hydroxy 1-nitro benzène
3-hydroxy 4-N-(2'aminoéthyl)amino 1-nitro benzène
2-N-(2'-hydroxyéthylamino) 4-hydroxy 1-nitro benzène.

Tous ces colorants peuvent être utilisés sous forme libre ou sous forme de sels tels que chlorhydrate, bromhydrate, sulfate et autres.

Ils peuvent être par ailleurs utilisés seuls ou en mélanges.

Le ou les colorants directs de formule (I) et/ou de formule (II) sont de préférence présents dans des concentrations allant de 0,01 à 10% en poids par rapport au poids total de la composition colorante.

En plus de la vapeur d'eau, le gaz vecteur peut contenir de la vapeur de solvant, des gaz tels que l'oxygène, l'azote, des mélange de gaz tels que l'air ou d'autres composés vaporisables.

Les solvants utilisables pour la production de vapeur sont des solvants organiques cosmétiquement acceptables et plus particulièrement des alcools tels que l'éthanol, l'isopropanol, l'alcool benzylique, l'alcool phényléthylique ou des glycols ou éthers de glycol tels que par exemple les éthers monométhylique, monoéthylique et monobutylique de l'éthylène glycol, le propylène glycol, le butylène glycol, le dipropylèneglycol ainsi que les alkyléthers comme le monobutyléther du diéthylèneglycol.

Le gaz comprend de préférence au moins 1% en volume de vapeur d'eau par rapport au volume total du gaz.

Le gaz est constitué de préférence soit uniquement ou essentiellement de vapeur d'eau, soit d'un mélange de vapeur d'eau et d'air.

La température du gaz est de préférence supérieure ou égale à 85°C et plus particulièrement comprise entre 85 et 150°C.

En particulier, le gaz est mis en contact avec la fibre à colorer pendant une durée allant de 0,01 seconde à 2 minutes.

De préférence, le gaz est mis en contact avec la fibre pendant une durée allant de 0,01 seconde à 30 secondes et encore plus préférentiellement de 1 à 10 secondes.
L'application du gaz peut être répétée plusieurs fois sur la même fibre, chaque opération se faisant selon la durée indiquée ci-dessus.

Dans un premier mode de mise en oeuvre du procédé selon l'invention qui est ici préféré, on applique sur les cheveux une composition de coloration capillaire contenant les colorants directs de formule (I) et/ou (II), puis on les soumet à l'action de la vapeur d'eau.

Selon d'autres modes de mise en oeuvre du procédé, il est également possible d'appliquer simultanément la composition colorante et le gaz comprenant de la vapeur d'eau.

Il est également possible de faire parvenir sur les cheveux tout ou partie de la composition colorante à l'aide du flux de gaz lorsque certains ou tous les constituants de la formule sont entraînables ou vaporisables.

Dans un mode particulier de réalisation de l'invention, l'application de vapeur d'eau est suivie d'un rinçage à l'eau.

La production d'un gaz chaud comprenant de la vapeur d'eau peut se faire à l'aide de tout appareil connu en soi. Toutefois, selon la présente invention, on utilise de préférence un appareil tel que celui décrit dans la demande de brevet français FR-A-2273492, ou tout autre appareil équivalent, qui convient particulièrement bien.

La composition tinctoriale utilisée dans le procédé selon l'invention peut se présenter sous des formes habituellement utilisées pour la teinture des cheveux telle que de liquide plus ou moins épaissi ou gélifié, de crème, de mousse en aérosol ou sous toute autre forme appropriée pour réaliser une teinture des cheveux.

Les compositions utilisées conformément à l'invention sont généralement des compositions aqueuses pouvant contenir des ingrédients habituellement utilisés dans les compositions cosmétiques destinées à la coloration des cheveux, tels que des solvants, des agents tensioactifs, des épaississants, des agents traitants, des agents alcalinisants ou acidifiants, des agents conservateurs, des parfums ou tout autre additif utilisé dans ce type de composition.

La composition tinctoriale contenant au moins un colorant direct de formule (I) et/ou (II) présente un pH généralement compris entre 2 et 11.

Les exemples qui suivent illustrent l'invention sans pour autant présenter un caractère limitatif.

### Exemple 1 (invention)

On utilise une composition présentant les caractéristiques suivantes :

| | |
|---|---|
| - 1-hydroxy 3-nitro 4-N-(2'-hydroxyéthyl)aminobenzène | 0,5 g |
| - Monométhyléther de propylène glycol | 10 g |
| - Diéthanolamide de coprah | 2 g |
| - Nonylphénol oxyéthyléné à 9 moles d'oxyde d'éthylène | 8 g |
| - 2-amino 2-méthyl 1-propanol qs | pH 9 |
| - Eau qsp | 100 g |

Le mode opératoire est le suivant : on applique la composition ci-dessus sur des cheveux naturels et sur les mêmes cheveux permanentés, on envoie ensuite deux jets de vapeur d'eau à 90°C pendant 30 secondes chacun. On rince à l'eau puis on sèche les cheveux.

On mesure ensuite la luminance L des deux types de cheveux traités à la vapeur (mesures effectuées au moyen d'un colorimètre MINOLTA CHROMA METER CR 200).

La différence : L naturels - L permanentés (ΔL), qui représente la sélectivité, est égale à 0,2. Cette valeur doit être la plus faible possible puisqu'elle représente la différence de couleur entre les deux mèches.

### Exemple 2 (comparatif)

On utilise la même composition que celle de l'exemple 1.

Le mode opératoire est le suivant : on applique la composition ci-dessus sur des cheveux naturels et sur des cheveux permanentés, on laisse poser pendant 30 minutes à température ambiante. On rince à l'eau puis on sèche les cheveux.

La différence : L naturels - L permanentés (ΔL), qui représente la sélectivité, est égale à 4,8 ; elle est beaucoup plus élevée que celle obtenue avec le procédé selon l'invention (ex. 1)

### Exemple 3 (invention)

On utilise une composition présentant les caractéristiques suivantes :

| | |
|---|---|
| - N,N',N'-tris(2-hydroxyéthyl) 1,4-diamino 2-nitro benzène | 0,5 g |
| - Monométhyléther de propylène glycol | 10 g |
| - Diéthanolamide de coprah | 8 g |
| - 2-amino 2-méthyl 1-propanol qs | pH 9 |
| - Eau qsp | 100 g |

Le mode opératoire est le suivant : on applique la composition ci-dessus sur des cheveux naturels et sur les mêmes cheveux permanentés, on envoie ensuite deux jets de vapeur d'eau à 90°C pendant 30 secondes chacun. On rince à l'eau puis on sèche les cheveux.

On mesure ensuite la luminance L des deux types de cheveux traités à la vapeur (mesures effectuées au moyen d'un colorimètre MINOLTA CHROMA METER CR 200).

La différence : L naturels - L permanentés (ΔL), qui représente la sélectivité, est égale à 5,6.

### Exemple 4 (comparatif)

On utilise la même composition que celle de l'exemple 3.

Le mode opératoire est le suivant : on applique la composition ci-dessus sur des cheveux naturels et sur des cheveux permanentés, on laisse poser pendant 30 minutes à température ambiante. On rince à l'eau puis on sèche les cheveux.

La différence : L naturels - L permanentés (ΔL), qui représente la sélectivité, est égale à 11,8 ; elle est deux fois plus élevée que celle obtenue avec le procédé selon l'invention (ex. 3)

### Exemple 5 (invention)

On utilise une composition présentant les caractéristiques suivantes :

| | |
|---|---|
| - 4-(N-éthyl N-2'hydroxyéthyl)amino 1-(2'-hydroxyéthyl)amino | |
| - 2-nitrobenzène | 0,5 g |
| - Monométhyléther de propylène glycol | 10 g |
| - Diéthanolamide de coprah | 2 g |
| - Nonylphénol oxyéthyléné à 9 moles d'oxyde d'éthylène | 8 g |
| - 2-amino 2-méthyl 1-propanol qs | pH 9 |
| - Eau qsp | 100 g |

Le mode opératoire est identique à celui de l'exemple 1.

On mesure ensuite la luminance L des deux types de cheveux traités à la vapeur (mesures effectuées au moyen d'un colorimètre MINOLTA CHROMA METER CR 200).

La différence : L naturels - L permanentés (ΔL), qui représente la sélectivité, est égale à 3,2.

### Exemple 6 (comparatif)

On utilise la même composition que celle de l'exemple 5.

Le mode opératoire est identique à celui de l'exemple 2.

On mesure ensuite la luminance L des deux types de cheveux (mesures effectuées au moyen d'un colorimètre MINOLTA CHROMA METER CR 200).

La différence : L naturels - L permanentés (ΔL), qui représente la sélectivité, est égale à 9,5 : elle est trois fois plus élevée que celle obtenue avec le procédé selon l'invention (ex. 5)

### Exemple 7 (invention)

On utilise une composition présentant les caractéristiques suivantes :

| | |
|---|---|
| - 1-hydroxy 2-amino 3-nitrobenzène | 0,5 g |
| - Monométhyléther de propylène glycol | 10 g |
| - Diéthanolamide de coprah | 2 g |
| - Nonylphénol oxyéthyléné à 9 moles d'oxyde d'éthylène | 8 g |
| - 2-amino 2-méthyl 1-propanol qs | pH 9 |
| - Eau qsp | 100 g |

Le mode opératoire est identique à celui de l'exemple 1.

On mesure ensuite la luminance L des deux types de cheveux traités à la vapeur (mesures effectuées au moyen d'un colorimètre MINOLTA CHROMA METER CR 200).

La différence : L naturels - L permanentés (ΔL), qui représente la sélectivité, est égale à 0,7.

### Exemple 8 (comparatif)

On utilise la même composition que celle de l'exemple 7.

Le mode opératoire est identique à celui de l'exemple 2.

On mesure la luminance L des deux types de cheveux (mesurées sur colorimètre MINOLTA CHROMA METER CR 200) .

La différence : L naturels - L permanentés (ΔL), qui représente la sélectivité, est égale à 2,7 : elle est beaucoup plus élevée que celle obtenue avec le procédé selon l'invention (ex. 7)

### Exemple 9 (comparatif)

On utilise une composition présentant les caractéristiques suivantes (non conforme à l'invention):

| | |
|---|---|
| - 2-nitro paraphénylènediamine | 0,5 g |
| - Monométhyléther de propylène glycol | 10 g |
| - Diéthanolamide de coprah | 2 g |
| - Nonylphénol oxyéthyléné à 9 moles d'oxyde d'éthylène | 8 g |
| - 2-amino 2-méthyl 1-propanol qs | pH 9 |
| - Eau qsp | 100 g |

On effectue la coloration d'une mèche de cheveux naturels et d'une mèche des mêmes cheveux permanentés selon le mode opératoire de l'exemple 1 (vapeur d'eau 90°C - 2 fois 30 secondes).

La différence : L naturels - L permanentés (ΔL), qui représente la sélectivité, est égale à 1,9.

On effectue ensuite, avec la même composition que ci-dessus, la coloration d'une mèche de cheveux naturels et d'une mèche des mêmes cheveux permanentés selon le mode opératoire de l'exemple 2 (Température ambiante - 30 minutes).

La différence : L naturels - L permanentés (ΔL), qui représente la sélectivité, est égale à 1,6.

Avec ce type de composition de coloration, qui n'entre pas dans le cadre de l'invention, la sélectivité n'est pas diminuée avec un traitement à la vapeur conforme à l'invention.

## Revendications

1. Procédé de teinture directe et non sélective des fibres kératiniques, caractérisé par le fait qu'il consiste à mettre en contact des fibres kératiniques sur lesquelles on a appliqué une composition contenant au moins un colorant direct choisi parmi les dérivés de la 2-nitroparaphénylènediamine de formule (I) ci-dessous et/ou les dérivés nitrés des aminophénols de formule (II) ci-dessous : 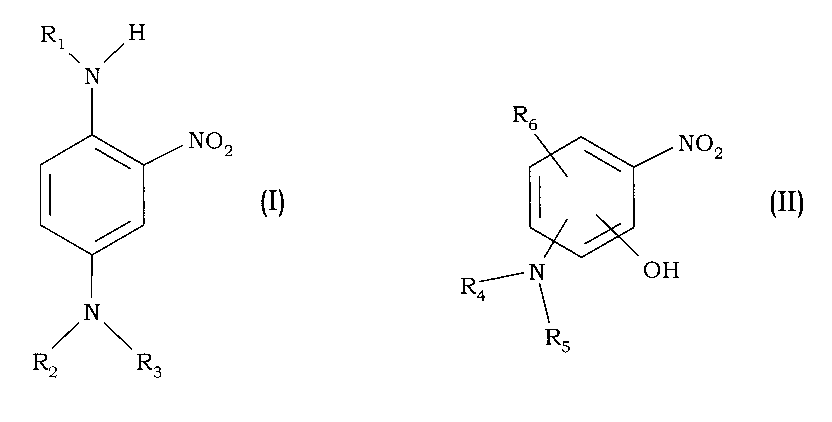 formules dans lesquelles :
R₁ est choisi parmi les radicaux alkyle en C₁-C₄, monohydroxyalkyle en C₂-C₄, polyhydroxyalkyle en C₂-C₄, les radicaux alkoxyalkyle et aminoalkyle en C₁-C₄ ;
R₂ et R₃ sont choisis parmi les radicaux alkyle en C₁-C₄, monohydroxyalkyle en C₂-C₄, polyhydroxyalkyle en C₂-C₄ ;
R₄ et R₅ sont choisis parmi l'hydrogène, les radicaux alkyle en C₁-C₄, monohydroxyalkyle en C₂-C₄, polyhydroxyalkyle en C₂-C₄ et aminoalkyle en C₁-C₄ ;
R₆ est choisi parmi l'hydrogène, les radicaux alkyle en C₁-C₄, les atomes d'halogène et le groupement NO₂.
avec un gaz contenant de la vapeur d'eau, la température dudit gaz étant supérieure à 75°C, le temps de contact entre ledit gaz et lesdites fibres à colorer étant inférieur à deux minutes.

2. Procédé selon la revendication 1, caractérisé par le fait que le gaz a une température supérieure ou égale à 85°C.

3. Procédé selon la revendication 2, caractérisé par le fait que le gaz a une température comprise entre 85 et 150°C.

4. Procédé selon l'une quelconque des revendications précédentes, caractérisé par le fait que le gaz est mis en contact avec la fibre à colorer pendant une durée allant de 0,01 seconde à 2 minutes.

5. Procédé selon la revendication 4, caractérisé par le fait que le gaz est mis en contact avec la fibre à colorer pendant une durée allant de 0,01 seconde à 30 secondes.

6. Procédé selon la revendication 5, caractérisé par le fait que le gaz est mis en contact avec la fibre à colorer pendant une durée allant de 1 seconde à 10 secondes.

7. Procédé selon l'une quelconque des revendications précédentes, caractérisé par le fait que l'application du gaz est répétée plusieurs fois sur une même fibre.

8. Procédé selon l'une quelconque des revendications précédentes, caractérisé par le fait que le gaz contient uniquement de la vapeur d'eau.

9. Procédé selon l'une quelconque des revendications 1 à 7, caractérisé par le fait que le gaz contient de la vapeur d'eau et au moins un autre composé sous forme de gaz ou de vapeur.

10. Procédé selon la revendication 9, caractérisé par le fait que le gaz contient de la vapeur d'eau et de l'air.

11. Procédé selon l'une quelconque des revendications précédentes, caractérisé par le fait que les colorants directs de formule (I) sont choisis parmi les composés suivants:
N,N',N'-tris(2'-hydroxyéthyl) 1,4-diamino 2-nitro benzène
1-méthylamino 2-nitro 4-bis(2'-hydroxyéthyl)amino benzène
1-méthylamino 2-nitro 4-(N-méthyl N-2'-hydroxyéthyl)amino benzène
1-(2'-aminoéthyl)amino 2-nitro 4-bis(2'-hydroxyéthyl)amino benzène
4-(N-éthyl N-2'-hydroxyéthyl)amino 1-(2'-hydroxyéthyl)amino 2-nitro benzène
1-(2'-méthoxyéthyl)amino 2-nitro 4-bis(2'-hydroxyéthyl)amino benzène
1-(2',3'-dihydroxypropyl)amino 2-nitro 4-bis(2'-hydroxyéthyl)amino benzène
1-(2',3'-dihydroxypropyl)amino 2-nitro 4-(N-méthyl N-2'-hydroxyéthyl)amino benzène
1-(2',3'-dihydroxypropyl)amino 2-nitro 4-(N-éthyl N-2'-hydroxyéthyl)amino benzène
1-méthylamino 2-nitro 4-(N-méthyl N-2',3'-dihydroxypropyl)amino benzène.

12. Procédé selon l'une quelconque des revendications précédentes, caractérisé par le fait que les colorants directs de formule (II) sont choisis parmi les composés suivants :
3-amino 4-hydroxy nitrobenzène
3-hydroxy 4-amino nitrobenzène
2-hydroxy 3-amino 1,5-dinitrobenzène
2-hydroxy 5-amino nitrobenzène
1-hydroxy 3-nitro 4-N-(2'-hydroxyéthyl)amino benzène
1-hydroxy 2-amino 3-nitro benzène
1-amino 2-nitro 4-hydroxy 5-méthyl benzène
1-N-(2'-hydroxyéthyl)amino 2-hydroxy 4-nitro benzène
1-hydroxy 2-N-(2'-hydroxyéthyl)amino 4,6-dinitrobenzène
2-chloro 6-N-éthylamino 1-hydroxy 4-nitro benzène
6-chloro 4-nitro 2-amino phénol
1-hydroxy 3-nitro 4-N-(3'-hydroxypropyl)amino benzène
1-hydroxy 2-N,N-(2'-hydroxyéthyl)amino 5-nitro benzène
2-amino 4-hydroxy 1-nitro benzène
2-hydroxy 4-amino 1-nitro benzène
1-hydroxy 3-nitro 4-amino benzène
2-hydroxy 5-N-(2'-hydroxyéthyl)amino 1-nitro benzène
2-hydroxy 6-amino 1-nitro benzène
2-hydroxy 3-amino 1-nitro benzène
2-hydroxy 3-chloro 6-amino 1-nitro benzène
2-N-(2'-hydroxyéthyl)amino 3-hydroxy 1-nitro benzène
3-N-(2'-hydroxyéthyl)amino 4-hydroxy 1-nitro benzène
2-hydroxy 6-N-(2'-hydroxyéthyl)amino 1-nitro benzène
1-hydroxy 2,6-dinitro 4-méthylamino benzène
2-N-(2'aminoéthyl)amino 4-hydroxy 1-nitro benzène
3-hydroxy 4-N-(2'aminoéthyl)amino 1-nitro benzène
2-N-(2'-hydroxyéthylamino) 4-hydroxy 1-nitro benzène.

13. Procédé selon l'une quelconque des revendications précédentes, caractérisé par le fait que le ou les colorants directs de formule (I) et/ou de formule (II) sont présents dans des concentrations allant de 0,01 à 10% en poids par rapport au poids total de la composition.

14. Procédé selon l'une quelconque des revendications précédentes, caractérisé par le fait que la composition contenant au moins un colorant direct présente un pH compris entre 2 et 11.

## Claims

1. Process for the direct and non-selective dyeing of keratinous fibres, characterized in that it consists in bringing keratinous fibres, to which has been applied a composition containing at least one direct dye chosen from the 2-nitro-para-phenylenediamine derivatives of formula (I) below and/or the nitro aminophenol derivatives of formula (II) below: 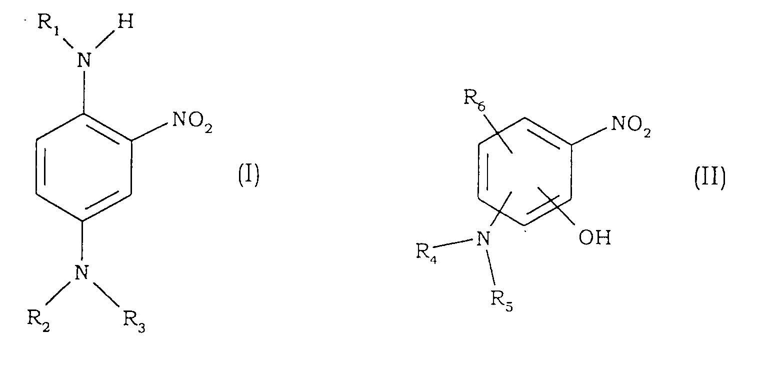 in which:
R₁ is chosen from C₁-C₄ alkyl, C₂-C₄ monohydroxyalkyl and C₂-C₄ polyhydroxyalkyl radicals and C₁-C₄ alkoxyalkyl and aminoalkyl radicals;
R₂ and R₃ are chosen from C₁-C₄ alkyl, C₂-C₄ monohydroxyalkyl and C₂-C₄ polyhydroxyalkyl radicals;
R₄ and R₅ are chosen from hydrogen and C₁-C₄ alkyl, C₂-C₄ monohydroxyalkyl, C₂-C₄ polyhydroxyalkyl and C₁-C₄ aminoalkyl radicals;
R₆ is chosen from hydrogen, C₁-C₄ alkyl radicals, halogen atoms and the NO₂ group,
into contact with a gas containing water vapour, the temperature of the said gas being greater than 75°C and the contact time between the said gas and the said fibres to be dyed being less than two minutes.

2. Process according to Claim 1, characterized in that the gas is at a temperature greater than or equal to 85°C.

3. Process according to Claim 2, characterized in that the gas is at a temperature between 85 and 150°C.

4. Process according to any one of the preceding claims, characterized in that the gas is brought into contact with the fibre to be dyed for a period ranging from 0.01 seconds to 2 minutes.

5. Process according to Claim 4, characterized in that the gas is brought into contact with the fibre to be dyed for a period ranging from 0.01 seconds to 30 seconds.

6. Process according to Claim 5, characterized in that the gas is brought into contact with the fibre to be dyed for a period ranging from 1 second to 10 seconds.

7. Process according to any one of the preceding claims, characterized in that application of the gas is repeated several times on the same fibre.

8. Process according to any one of the preceding claims, characterized in that the gas exclusively contains water vapour.

9. Process according to any one of Claims 1 to 7, characterized in that the gas contains water vapour and at least one other compound in gas or vapour form.

10. Process according to Claim 9, characterized in that the gas contains water vapour and air.

11. Process according to any one of the preceding claims, characterized in that the direct dyes of formula (I) are chosen from the following compounds:
N,N',N'-tris(2'-hydroxyethyl)-1,4-diamino-2-nitrobenzene
1-methylamino-2-nitro-4-bis(2'-hydroxyethyl)aminobenzene
1-methylamino-2-nitro-4-(N-methyl-N-2'-hydroxyethyl)-aminobenzene
1-(2'-aminoethyl)amino-2-nitro-4-bis(2'-hydroxyethyl)-aminobenzene
4-(N-ethyl-N-2'-hydroxyethyl)amino-1-(2'-hydroxyethyl)-amino-2-nitrobenzene
1-(2'-methoxyethyl)amino-2-nitro-4-bis(2'-hydroxyethyl)-aminobenzene
1-(2',3'-dihydroxypropyl)amino-2-nitro-4-bis(2'-hydroxyethyl)aminobenzene
1-(2',3'-dihydroxypropyl)amino-2-nitro-4-(N-methyl-N-2'-hydroxyethyl)aminobenzene
1-(2',3'-dihydroxypropyl)amino-2-nitro-4-(N-ethyl-N-2'-hydroxyethyl)aminobenzene
1-methylamino-2-nitro-4-(N-methyl-N-2',3'-dihydroxypropyl)aminobenzene.

12. Process according to any one of the preceding claims, characterized in that the direct dyes of formula (II) are chosen from the following compounds:
3-amino-4-hydroxynitrobenzene
3-hydroxy-4-aminonitrobenzene
2-hydroxy-3-amino-1,5-dinitrobenzene
2-hydroxy-5-aminonitrobenzene
1-hydroxy-3-nitro-4-N-(2'-hydroxyethyl)aminobenzene
1-hydroxy-2-amino-3-nitrobenzene
1-amino-2-nitro-4-hydroxy-5-methylbenzene
1-N-(2'-hydroxyethyl)amino-2-hydroxy-4-nitrobenzene
1-hydroxy-2-N-(2'-hydroxyethyl)amino-4,6-dinitrobenzene
2-chloro-6-N-ethylamino-1-hydroxy-4-nitrobenzene
6-chloro-4-nitro-2-aminophenol
1-hydroxy-3-nitro-4-N-(3'-hydroxypropyl)aminobenzene
1-hydroxy-2-N,N-(2'-hydroxyethyl)amino-5-nitrobenzene
2-amino-4-hydroxy-1-nitrobenzene
2-hydroxy-4-amino-1-nitrobenzene
1-hydroxy-3-nitro-4-aminobenzene
2-hydroxy-5-N-(2'-hydroxyethyl)amino-1-nitrobenzene
2-hydroxy-6-amino-1-nitrobenzene
2-hydroxy-3-amino-1-nitrobenzene
2-hydroxy-3-chloro-6-amino-1-nitrobenzene
2-N-(2'-hydroxyethyl)amino-3-hydroxy-1-nitrobenzene
3-N-(2'-hydroxyethyl)amino-4-hydroxy-1-nitrobenzene
2-hydroxy-6-N-(2'-hydroxyethyl)amino-1-nitrobenzene
1-hydroxy-2,6-dinitro-4-methylaminobenzene
2-N-(2'aminoethyl)amino-4-hydroxy-1-nitrobenzene
3-hydroxy-4-N-(2'aminoethyl)amino-1-nitrobenzene
2-N-(2'-hydroxyethylamino)-4-hydroxy-1-nitrobenzene.

13. Process according to any one of the preceding claims, characterized in that the direct dye or dyes of formula (I) and/or of formula (II) are present in concentrations ranging from 0.01 to 10 % by weight relative to the total weight of the composition.

14. Process according to any one of the preceding claims, characterized in that the composition containing at least one direct dye has a pH between 2 and 11.

## Patentansprüche

1. Verfahren zur direkten und nicht-selektiven Färbung von Keratinfasern, dadurch gekennzeichnet, daß man Keratinfasern, auf die eine Zusammensetzung mit einem Gehalt an mindestens einem Direktfarbstoff, das unter Derivaten von 2-Nitro-p-phenylendiamin der Formel (I) und/oder von Nitroaminophenolderivaten der Formel (II) ausgewählt ist, aufgebracht ist, 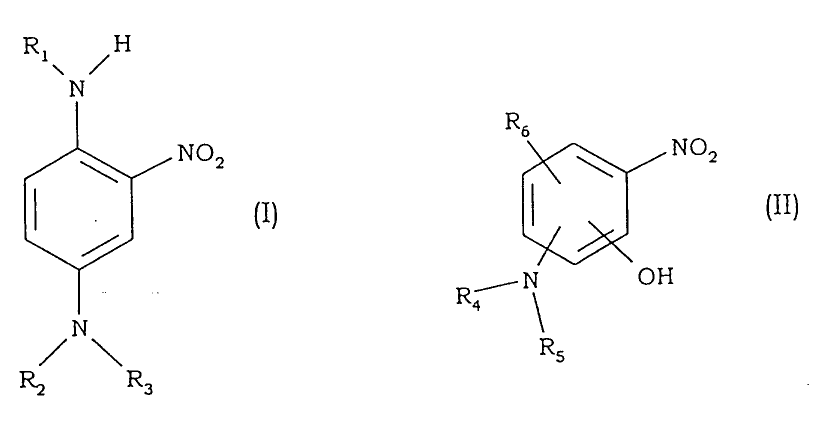 worin:
R₁ unter C₁-C₄-Alkylresten, C₂-C₄-Monohydroxyalkylresten, C₂-C₄-Polyhydroxyalkylresten, C₁-C₄-Alkoxyalkyl- und -Aminoalkylresten ausgewählt ist;
R₂ und R₃ unter C₁-C₄-Alkylresten, C₂-C₄-Monohydroxyalkylresten und C₂-C₄-Polyhydroxyalkylresten ausgewählt sind;
R₄ und R₅ unter Wasserstoff, C₁-C₄-Alkylresten, C₂-C₄-Monohydroxyalkylresten, C₂-C₄-Polyhydroxyalkylresten und C₁-C₄-Aminoalkylresten ausgewählt sind; und
R₆ unter Wasserstoff, C₁-C₄-Alkylresten, Halogenatomen und der NO₂-Gruppe ausgewählt ist.
mit einem Wasserdampf enthaltenden Gas in Kontakt bringt, wobei die Temperatur des Gases über 75°C liegt und die Kontaktzeit zwischen dem Gas und den zu färbenden Fasern weniger als 2 Minuten beträgt.

2. Verfahren nach Anspruch 1, dadurch gekennzeichnet, daß das Gas eine Temperatur von 85°C oder mehr aufweist.

3. Verfahren nach Anspruch 2, dadurch gekennzeichnet, daß das Gas eine Temperatur von 85°C bis 150°C aufweist.

4. Verfahren nach einem der vorstehenden Ansprüche, dadurch gekennzeichnet, daß das Gas mit der zu färbenden Faser für eine Zeitspanne von 0,01 Sekunden bis 2 Minuten in Kontakt gebracht wird.

5. Verfahren nach Anspruch 4, dadurch gekennzeichnet, daß das Gas mit der zu färbenden Faser für eine Zeitspanne von 0,01 Sekunden bis 30 Sekunden in Kontakt gebracht wird.

6. Verfahren nach Anspruch 5, dadurch gekennzeichnet, daß das Gas mit der zu färbenden Faser für eine Zeitspanne von 1 bis 10 Sekunden in Kontakt gebracht wird.

7. Verfahren nach einem der vorstehenden Ansprüche, dadurch gekennzeichnet, daß man das Gas mehrfach auf die gleiche Faser einwirken läßt.

8. Verfahren nach einem der vorstehenden Ansprüche, dadurch gekennzeichnet, daß das Gas nur Wasserdampf enthält.

9. Verfahren nach einem der Ansprüche 1 bis 7, dadurch gekennzeichnet, daß das Gas Wasserdampf und mindestens eine weitere Verbindung in Form eines Gases oder Dampfes enthält.

10. Verfahren nach Anspruch 9, dadurch gekennzeichnet, daß das Gas Wasserdampf und Luft enthält.

11. Verfahren nach einem der vorstehenden Ansprüche, dadurch gekennzeichnet, daß die Direktfarbstoffe der Formel (I) unter folgenden Verbindungen ausgewählt sind:
N,N',N'-Tris-(2'-hydroxyethyl)-1,4-diamino-2-nitrobenzol,
1-Methylamino-2-nitro-4-bis-(2'-hydroxyethyl)-aminobenzol,
1-Methylamino-2-nitro-4-(N-methyl-N-2'-hydroxyethyl)-aminobenzol,
1-(2'-Aminoethyl)-amino-2-nitro-4-bis-(2'-hydroxyethyl)-aminobenzol,
4-(N-Ethyl-N-2'-hydroxyethyl)-amino-1-(2'-hydroxyethyl)-amino-2-nitrobenzol,
1-(2'-Methoxyethyl)-amino-2-nitro-4-bis-(2'-hydroxyethyl)-aminobenzol,
1-(2',3'-Dihydroxypropyl)-amino-2-nitro-4-bis-(2'-hydroxyethyl)-aminobenzol,
1-(2',3'-Dihydroxypropyl)-amino-2-nitro-4-(N-methyl-N-2'-hydroxyethyl)-aminobenzol,
1-(2',3'-Dihydroxypropyl)-amino-2-nitro-4-(N-ethyl-N-2'-hydroxyethyl)-aminobenzol und
1-Methylamino-2-nitro-4-(N-methyl-N-2',3'-dihydroxypropyl)-aminobenzol.

12. Verfahren nach einem der vorstehenden Ansprüche, dadurch gekennzeichnet, daß die Direktfarbstoffe der Formel (II) unter folgenden Verbindungen ausgewählt sind:
3-Amino-4-hydroxy-nitrobenzol,
3-Hydroxy-4-amino-nitrobenzol,
2-Hydroxy-3-amino-1,5-dinitrobenzol,
2-Hydroxy-5-amino-nitrobenzol,
1-Hydroxy-3-nitro-4-N-(2'-hydroxyethyl)-aminobenzol,
1-Hydroxy-2-amino-3-nitrobenzol,
1-Amino-2-nitro-4-hydroxy-5-methylbenzol,
1-N-(2'-Hydroxyethyl)-amino-2-hydroxy-4-nitrobenzol,
1-Hydroxy-2-N-(2'-hydroxyethyl)-amino-4,6-dinitrobenzol,
2-Chlor-6-N-ethylamino-1-hydroxy-4-nitrobenzol,
6-Chlor-4-nitro-2-aminophenol,
1-Hydroxy-3-nitro-4-N-(3'-hydroxypropyl)-aminobenzol,
1-Hydroxy-2-N,N-(2'-hydroxyethyl)-amino-5-nitrobenzol,
2-Amino-4-hydroxy-1-nitrobenzol,
2-Hydroxy-4-amino-1-nitrobenzol,
1-Hydroxy-3-nitro-4-aminobenzol,
2-Hydroxy-5-N-(2'-hydroxyethyl)-amino-1-nitrobenzol,
2-Hydroxy-6-amino-1-nitrobenzol,
2-Hydroxy-3-amino-1-nitrobenzol,
2-Hydroxy-3-chlor-6-amino-1-nitrobenzol,
2-N-(2'-Hydroxyethyl)-amino-3-hydroxy-1-nitrobenzol,
3-N-(2'-Hydroxyethyl)-amino-4-hydroxy-1-nitrobenzol,
2-Hydroxy-6-N-(2'-hydroxyethyl)-amino-1-nitrobenzol,
1-Hydroxy-2,6-dinitro-4-methylaminobenzol,
2-N-(2'-Aminoethyl)-amino-4-hydroxy-1-nitrobenzol,
3-Hydroxy-4-N-(2'-aminoethyl)-amino-1-nitrobenzol und
2-N-(2'-Hydroxyethylamino)-4-hydroxy-1-nitrobenzol.

13. Verfahren nach einem der vorstehenden Ansprüche, dadurch gekennzeichnet, daß das oder die Direktfarbstoffe der Formel (I) und/oder der Formel (II) in Konzentrationen von 0,01 bis 10 Gew.-%, bezogen auf das Gewicht der gesamten Zusammensetzung, vorhanden sind.

14. Verfahren nach einem der vorstehenden Ansprüche, dadurch gekennzeichnet, daß die Zusammensetzung, die mindestens einen Direktfarbstoff enthält, einen pH-Wert von 2 bis 11 aufweist.
